# EUROPÄISCHE PATENTSCHRIFT

(11) **EP 2 573 164 B1**
(45) Veröffentlichungstag und Bekanntmachung des Hinweises auf die Patenterteilung: **04.01.2017**
(21) Anmeldenummer: 12006677.4
(22) Anmeldetag: 24.09.2012
(51) Int. Cl.: C12N 1/14, C12N 1/26, C12N 11/10, C02F 3/10, C02F 3/34, B09C 1/10

(54) **Verfahren zum mikrobiologischen Abbau von Kohlenwasserstoffen**
Method for the microbiological degeneration of hydrocarbons
Procédé de décomposition microbiologique d'hydrocarbures

(30) Priorität: 23.09.2011 DE 102011114140
(43) Veröffentlichungstag der Anmeldung: 27.03.2013
(73) Patentinhaber: Horstmann Maschinenbau GmbH, 48619 Heek (DE)
(72) Erfinder: Dettmann, Peter, 48431 Rheine (DE); Ebeling, Norbert, 48612 Horstmar (DE); Büttner, Hermann, 48565 Steinfurt (DE); Müller, Jürgen, 48249 Dülmen (DE); Doedt, Sebastian, 48629 Metelen (DE); Bosse, Tobias, 49479 Ibbenbüren (DE); Böckers, Rudolf, 48619 Heek (DE)
(74) Vertreter: Habbel, Ludwig

(56) Entgegenhaltungen:
- WO-A1-93/17971
- WO-A1-2008/095589
- UA-U- 23 917
- DATABASE BIOSIS [Online] BIOSCIENCES INFORMATION SERVICE, PHILADELPHIA, PA, US; 1977, KOVAL E Z ET AL: "CLADOSPORIUM-RESINAE AND ITS DEGRADATION OF GASOLINE AND OTHER PETROLEUM PRODUCTS", XP002688521, Database accession no. PREV197866014177
- OH KI-BONG ET AL: "Biodegradation of hydrocarbons by an organic solvent-tolerant fungus, Cladosporium resinae NK-1", JOURNAL OF MICROBIOLOGY AND BIOTECHNOLOGY, Bd. 11, Nr. 1, Februar 2001 (2001-02), Seiten 56-60, XP008158524, ISSN: 1017-7825
- FRÉDÉRIC CHAILLAN ET AL: "Identification and biodegradation potential of tropical aerobic hydrocarbon-degrading microorganisms", RESEARCH IN MICROBIOLOGY, Bd. 155, Nr. 7, 1. September 2004 (2004-09-01), Seiten 587-595, XP055046309, ISSN: 0923-2508, DOI: 10.1016/j.resmic.2004.04.006
- B. QI ET AL: "Biodegradation of volatile organic compounds by five fungal species", APPLIED MICROBIOLOGY AND BIOTECHNOLOGY, Bd. 58, Nr. 5, 1. April 2002 (2002-04-01), Seiten 684-689, XP055046312, ISSN: 0175-7598, DOI: 10.1007/s00253-002-0938-3
- KOCH B ET AL: "SAND AND ACTIVATED CARBON AS BIOFILM CARRIERS FOR MICROBIAL DEGRADATION OF PHENOLS AND NITROGEN-CONTAINING AROMATIC COMPOUNDS", WATER RESEARCH, ELSEVIER, AMSTERDAM, NL, Bd. 25, Nr. 1, 1. Januar 1991 (1991-01-01) , Seiten 1-08, XP000175063, ISSN: 0043-1354, DOI: 10.1016/0043-1354(91)90091-4
- DATABASE WPI Week 199414 Thomson Scientific, London, GB; AN 1994-111949 XP002688522, & JP 6 056616 A (KANKYO RYOKUKA SHIGEN KAIHATSU CENT KK) 1. März 1994 (1994-03-01)
- FÁTIMA M. BENTO ET AL: "Biodeterioration of stored diesel oil: studies in Brazil", INTERNATIONAL BIODETERIORATION & BIODEGRADATION, Bd. 47, Nr. 2, 1. März 2001 (2001-03-01), Seiten 107-112, XP055046282, ISSN: 0964-8305, DOI: 10.1016/S0964-8305(00)00112-8
- SEIFERT K A ET AL: "Taxonomy, nomenclature and phylogeny of three cladosporium-like hyphomycetes, Sorocybe resinae, Seifertia azaleae and the Hormoconis anamorph of Amorphotheca resinae", STUDIES IN MYCOLOGY, Nr. 58, 2007, Seiten 235-245, XP002688523, ISSN: 0166-0616

## Beschreibung

Die Erfindung betrifft ein Verfahren zum mikrobiologischen Abbau unerwünschter Anteile von wasser- und erdreichgefährdenden Mineralölen und Mineralölprodukten, im Folgenden "Kohlenwasserstoffe" genannt, wie limnischen und marinen Gewässern, hauptsächlich aus Wasser bestehenden Flüssigkeiten, Böden, Sedimenten, Schlämmen, Abfallgemischen und ähnlichen Substraten, wobei auf das und/oder in das jeweilige Substrat zum Abbau der in ihm enthaltenen Kohlenwasserstoffe ein festes Material, bestehend aus einem die Kohlenwasserstoffe kontaktierenden kohlenstoffhaltigen Träger mit biogenetischer Eigenschaft zur Ernährung von kohlenwasserstoffabbauenden Fungus-Arten auf- bzw. eingebracht wird, und das Material mit zur Degradierung von Kohlenwasserstoffen befähigten Fungi besetzt wird.

Durch hydrothermale Carbonisierung aus Biomassen hergestellten Biokohle eignet sich zur Förderung des selektiven Wachstums von Pilzen der Familie *Amorphothecaceae* in und auf Substraten, die in Kontakt mit der Biokohle stehen. Die Biokohle wiederum ist dadurch ausgezeichnet, dass durch ihre Struktur, Nährstoffgehalt, Mineraliengehalt und/oder geeignete pH-Bereiche das Wachstum der Pilze der Familie *Amorphothecaceae* in besonderer Weise gefördert wird.

Es wurde gefunden, dass die vorgenannte Biokohle mit den Pilzen zum mikrobiologischen Abbau unerwünschter Anteile von wasser- und erdreichgefährdenden Mineralölen und Mineralölprodukten geeignet ist. Zu diesen Produkten zählen die genannten Kohlenwasserstoffe, also Rohöle, Schweröle, Heizöle, Dieseltreibstoffe, Ottotreibstoffe, Motoröle, Schmiermittel und Hydraulikflüssigkeiten in limnischen und marinen Gewässern, hauptsächlich aus Wasser bestehenden Flüssigkeiten, Böden, Sedimenten, Schlämmen, Abfallgemischen und ähnlichen Substraten, wobei auf das und/oder in das jeweilige Substrat zum Abbau der in ihm enthaltenen Kohlenwasserstoffe Kulturen von Mikroorganismen zur Vermehrung eingebracht werden, die zum Abbau der Kohlenwasserstoffe befähigt sind, wobei Biokohle sowohl Träger der Mikroorganismen als auch ein Beschleuniger des Wachstums ist.

Aus den folgenden Druckschriften sind jeweils Verfahren zum mikrobiologischen Abbau von Kohlenwasserstoffen mit Hilfe *von Amorphotheca resinae* / *Cladosporium resinae* bekannt:
1. DATABASE BIOSIS [Online] BIOSCIENCES INFORMATION SERVICE, PHILADELPHIA, PA, US; 1977, KOVAL E Z ET AL: "CLADOSPORIUM-RESINAE AND ITS DEGRADATION OF GASOLINE AND OTHER PETROLEUM PRODUCTS"
2. OH KI-BONG ET AL: "Biodegradation of hydrocarbons by an organic solvent-tolerant fungus, Cladosporium resinae NK-1", JOURNAL OF MICROBIOLOGY AND BIOTECHNOLOGY, Bd. 11, Nr. 1, Februar 2001, Seiten 56-60
3. FRÉDÉRIC CHAILLAN ET AL: "Identification and biodegradation potential of tropical aerobic hydrocarbon-degrading microorganisms", RESEARCH IN MICROBIOLOGY, Bd. 155, Nr. 7, 1. September 2004, Seiten 587-595
4. B. QI ET AL: "Biodegradation of volatile organic compounds by five fungal species", APPLIED MICROBIOLOGY AND BIOTECHNOLOGY, Bd. 58, Nr. 5, 1. April 2002, Seiten 684-689
5. WO 93/17971 A1

Aus den folgenden Druckschriften ist jeweils die Anwendung von makroporöser Kohle und aktivierter Kohle als Träger für Mikroorganismen in Verfahren zum mikrobiologischen Abbau von Kohlenwasserstoffen und / oder organischen, aromatischen Verbindungen, oder zum Desinfizieren von Erde bekannt:
1. UA 23 917 U
2. KOCH B ET AL: "SAND AND ACTIVATED CARBON AS BIOFILM CARRIERS FOR MICROBIAL DEGRADATION OF PHENOLS AND NITROGEN-CONTAINING AROMATIC COMPOUNDS", WATER RESEARCH, Bd. 25, Nr. 1, 1. Januar 1991 (1991-01-01), Seiten 1-8
3. DATABASE WPI Week 199414 Thomson Scientific, London, GB; AN 1994-111949 & JP 6 056616 A Es ist bekannt, dass Mikroorganismen in der Lage sind, umweltschädliche Stoffe in Böden und Gewässern abzubauen, wobei als Endprodukte vorzugsweise Wasser und Kohlendioxid entstehen. Dabei sollen das Gefüge und die Flora und Fauna des kontaminierten Bodens weitgehend intakt bleiben. Eine zusammenfassende Betrachtung angewandter Techniken des mikrobiologischen Abbaus findet sich in der Schrift »Mikrobiologische Verfahren bei der Altlastsanierung«, herausgegeben von der Landesanstalt für Umweltschutz Baden-Württemberg, Karlsruhe, 1996. Hier sei insbesondere auf das XENEX-Verfahren (Seite 29/30 der Schrift) verwiesen. Gemäß dem genannten Verfahren werden sogenannte Biobeete erstellt, die mittels spezieller Melioratoren zusammen mit den Schadstoff belasteten Böden in situ bearbeitet und damit biologisch rekultiviert werden.

Als weiterer Stand der Technik sei DE 103 08 170 A1 genannt. Hier wird ein Verfahren beschrieben, bei dem unter Verwendung einer wässrigen Proteinlösung zur Kultivierung eines Pilzes aus der Gattung *Fusarium* umweltbelastende und giftige Stoffe, die organische Lösungsmittel enthalten, durch den Pilz abgebaut werden. Kontaminiertes Erdreich muss nicht abgetragen werden, da der Pilz zur natürlichen Bodenflora gehört. Die Pilze der Gattung Fusarium sollen in der Lage sein, auch in Vielkomponentengemischen, die zum Beispiel Dieselkraftstoff, Motorenöl, Erdöl oder Heizöl enthalten, zu wachsen, ihren Stoffwechsel zu betreiben, sich zu vermehren und die in den Gemischen enthalte- nen verschiedenen Kohlenwasserstoffe abzubauen. Eine Gewinnung von ausreichenden Mengen an Pilzkulturen scheint jedoch schwierig zu sein.

Es stellt sich die Aufgabe, eine Kombination von geeignetem Träger und darauf wachsenden Kohlenwasserstoffe abbauenden Pilzen vorzuschlagen, die wirtschaftlich und ökologisch neutral in Ökosystemen einsetzbar ist. Diese Aufgabe wird durch ein Verfahren der eingangs genannten Art gelöst, bei dem als Träger an sich bekannte Biokohle verwendet wird, die durch katalytische hydrothermale Carbonisierung gewonnen wurde, und als degradierende Fungi Pilze von der Familie *Amorphothecaceae* gewählt werden, wobei Pilzarten dieser Familie als Bestandteil der natürlichen Flora die in Kontakt mit dem Substrat zu bringende Biokohle besiedeln und/oder in Form einer Pilzkultur der Biokohle hinzugefügt werden.

Pilze der Familie *Amorphothecaceae* sind in der Lage, durch Bildung von Oxygenasen Kohlenwasserstoffe, einschließlich cyclischer Kohlenwasserstoffe, zu degradieren und letztendlich zu Wasser und CO₂ abzubauen. Mit mit diesen Pilzen versehene Biokohle ist der Abbau von umweltschädlichen Mineralölen und Mineralölprodukten in Böden und Gewässern, gegebenenfalls auch in Luft, möglich, wobei der Abbau vorzugsweise in situ, erforderlichenfalls auch ex situ durchführbar ist. Der mikrobiologische Abbau der kontaminierenden Substanz, also des Kontaminenten, kann unter vertretbarem energetischem und technischem Aufwand und unter Einsatz von Stoffen, die das zu reinigende Substrat intakt lassen, erfolgen. Dabei ist zu berücksichtigen, dass eine große Mannigfaltigkeit derartiger Mineralöle und Mineralölprodukte besteht.

Als zum Abbau befähigte Mikroorganismen sind Kohlenwasserstoffe degradierende Pilze von der Familie *Amorphothecaceae* gewählt, wobei Pilzarten dieser Familie als Bestandteil der natürlichen Flora die in Kontakt mit dem Substrat stehende Biokohle besiedeln oder in Form einer Pilzkultur der Biokohle hinzugefügt werden, die als Träger auf Biokohle, die durch hydrothermale Carbonisierung gewonnen wurde, in Kontakt mit dem Substrat gesetzt werden.

Durch hydrothermale Karbonisierung hergestellte Biokohle wird beispielsweise beschrieben in der WO 2008/095589 A1. Ausgehend von einem Gemisch aus Biomasse und Wasser und Beigabe von wenigstens einem Katalysator - insbesondere wird hier Zitronensäure genannt - lässt sich die Masse durch Temperatur- und/oder Druckerhöhung in Biokohle umwandeln. Der Begriff "Biomasse" umfasst im Sinne der Erfindung gemäß WO 2008/095589 A1 sämtliche lebenden, toten und/oder zersetzten Organismen. Dazu zählen neben Pflanzen insbesondere auch Abfall und/oder Restholz, Stroh, Gras, Dung, Laub, Klärschlamm und/oder organischer Hausmüll. Die Biomasse kann dabei in unterschiedlichen Zusammensetzungen, Qualitäten, Größen, und/oder dergleichen Verwendung finden, je nach Bedarf und gewünschtem Umwandlungsprodukt.

Dem Fachmann ist demnach bekannt, dass trotz Ähnlichkeit der Beschaffenheit in Biokohle Unterschiede je nach Ausgangs-Biomasse erhalten sein können.

Vor dem Einbringen der Biokohle kann eine Eignungsfeststellung der zu verwendenden Substanzen anhand einer charakteristischen Probenmenge des mit einem oder mehreren Kohlenwasserstoffen verseuchten Substrates mit Biokohle und einer Ansatzmenge an degradierenden Pizen und/oder deren Sporen angesetzt werden und die Vermehrung der Pilzsubstanz unter den zu erwartenden Umgebungsparametern, wie Temperatur, Kohlenwasserstoff-Konzentration und pH-Wert, beobachtet werden. Nach beobachteter Vermehrung der Pilzsubstanz erfolgt ein unter gleichen Bedingungen stehender Großansatz mit dem Substrat.

Die Kontaminenten, wobei hier Roh-, Schwer- und Heizöle, sowie Kfz-Treibstoffe, nämlich Benzin und Dieselöle, an erster Stelle zu nennen sind, sind mit der als Träger der Mikroorganismen genannten Biokohle in Kontakt zu bringen, die die Kontaminenten in einer ersten Wirkungsstufe adsorbiert. In einer zweiten Stufe werden die Kontaminenten mikrobiologisch durch die zum Abbau befähigten Mikroorganismen degradiert.

Die Erfindung bedient sich also einer Biokohle, die angereichert ist mit Pilzen oder Pilzsporen der Familie Amorphothecaceae, wobei diese Biokohle zur Durchführung des Verfahrens geeignet ist. Die Biokohle zur Verwendung gemäß Erfindung zeichnet sich dadurch aus, dass mit ihr durch Struktur, Nährstoffgehalt, Mineraliengehalt und/oder geeignete pH-Bereiche das Wachstum der Pilze der Familie Amorphothecaceae katalysierbar ist.

Unter Biokohle soll hydrothermal karbonisierte Biokohle verstanden werden. Zur Herstellung dieser Biokohle wird Biomasse mit einem Katalysator-Zusatz zur Reaktionsbeschleunigung in einen Druckbehälter gegeben und auf etwa 180°C erhitzt. Nach einer Reaktionszeit von etwa 12 Stunden oder länger entsteht Biokohle mit einer torf- bis koksartigen Struktur. Hierzu finden sich bei Friedrich Bergius, "Chemical reactions under high pressure", Vortrag gehalten zum Anlass der Verleihung des Nobel-Preise, 21.5.1932 , weitere Angaben.

In der Patentliteratur werden Verfahrensabläufe zur Herstellung von Biokohle beschrieben; genannt sei hier als Beispiel die EP 2 166 061. Es wird davon ausgegangen, dass bei unterschiedlichen Ausgangssubstanzen und Verfahrensabläufen auch voneinander abweichende Konsistenzen der Biokohle erzeugt werden, von denen nicht alle zur Durchführung des im Folgenden beschriebenen Verfahrens geeignet sind. Durch die vorgeschaltete Analyse an einer kleinen Menge lässt sich ein geeignetes symbiotisches System von Biokohle und Pilzen der Familie Amorphothecaceae ermitteln. Dabei ist natürlich nicht ausgeschlossen, dass eine sol- che Konfiguration für bestimmte Kontaminenten beziehungsweise Kontaminentdosierungen ungeeignet ist, zu dass das Verfahren in Sonderfällen nicht angewendet werden kann.

Die zu verwendende Biokohle kann aus einer Komponente bestehen oder aus mehreren Komponenten gemischt sein. Es sollte jeweils ein hohes Adsorptionsvermögen gezeigt werden. Biokohle und Pilze bestimmter Arten bilden eine Art symbiotische Gemeinschaft. Dabei sind Pilze der Familie *Amorphothecaceae* mit Vorteil geeignet, hierbei vorzugsweise die Gattung *Amorphoteca,* mit der Art *Amorphotheca resinae,* die synonym auch als *Hormoconis resi*nae bezeichnet wird, einzusetzen ist, da diese Kohlenwasserstoffe der eingangs genannten Art degradieren können.

Die letztgenannte Spezies ist auch als »Dieselpilz« bekannt und wird beispielsweise in der Dissertation von H.M. FÜRST »Ökologie des Hyphenpilzes *Hormoconis resinae* und Eigenschaften seines n-Alkan-induzierten P450-Monooxygenasesystems« beschrieben (Erscheinungsjahr 2000).

Weitere geeignete Arten der Familie *Amorphothecaceae* sind:
- *Amorphotheca resinae* in seinem perfect state und/oder in seinem imperfect state und/oder in seinem Intermediat,
- *Cladosporium resinae, auch asexuelle Formen wie*
- *Cladosporium resinae f. avellaneum,*
- *Cladosporium resinae f. resiane (= f. viride),*
- *Cladosporium resinae f. albidum,*
- *Cladosporium resinae f. sterile*
- *Homodendrum resinae,*
- *Hormoconis resinae,*
- *Cladosporium avellaneum.*

Pilze dieser Arten besiedeln als Bestandteil der natürlichen Flora die in Kontakt mit dem Substrat stehende Biokohle oder werden in Form einer Pilzkultur oder als Sporen der Biokohle hinzugefügt.

Wird das Verfahren in situ, d.h. nach Überführung der Biokohle und der Pilzkulturen in das verunreinigte Substrat vor Ort ausgeführt, so sollte dies unter empirisch ermittelten optimierten Bedingungen, vorzugsweise bei einer Temperatur von 5° bis 45°C, durchgeführt werden.

Die durch hydrothermale Carbonisierung aus Biomassen hergestellte Biokohle kann auch mit Kohlenwasserstoffe degradierenden Pilz von der Art *Cladosporium resinae* versehen sein. Bei einem in situ Verfahren erfolgt eine Überführung der Biokohle und der Pilzkulturen in das verunreinigte Substrat vor Ort unter empirisch ermittelten optimierten Bedingungen, vorzugsweise bei einer Temperatur von 5°C bis 45°C. Mineralöl- und/oder Mineralölproduktbestandteile werden durch den Pilz verstoffwechselt.

Auch wenn das Verfahren ex situ, d.h. nach Überführung des verunreinigten Substrates in einen Reaktor zusammen mit Biokohle und Pilzkulturen ausgeführt wird, sollte dies unter empirisch ermittelten optimierten Bedingungen, vorzugsweise bei einer Temperatur von 5° bis 45°C, geschehen.

Vorzugsweise sollte die Biokohle mit einer die Kohlenwasserstoffe adsorbierenden porösen Mikrostruktur ausgestattet sein.

Zur Feststellung der Geeignetheit zur Verwendung der Biokohle wird eine Probe von 5g granulierter Biokohle mit 5ml Dieseltreibstoff und der gleichen Menge Wasser versetzt, mit Sporen von Pilzen der Familie *Amorphothecaceae* geimpft, die Probe aerob bei einem pH-Wert von 6,5 bis 7,2 bei 30° C inkubiert; die Anzahl der gebildeten Pilzkolonien dient als Gradmesser für die Geeignetheit der Biokohle.

Als die die Kohlenwasserstoffe degradierenden Pilze werden auch solche der Familie Amorphothecaceae von der Gattung Amorphoteca angesiedelt, wobei Pilze der Familie Amorphothecaceae als Bestandteil des natürlichen Ökosystems die in Kontakt mit dem Substrat stehende Biokohle besiedeln oder in Form einer Pilzkultur der Biokohle hinzugefügt werden.

Der mit der Biokohle verbundene, die Kohlenwasserstoffe degradierende Pilz kann wahlweise von der Art *Amorphotheca resinae* in seinem perfect state und/oder in seinem imperfect state und/oder in seinem Intermediat, hier im Speziellen benannt die Synonyme creosote fungus, *Cladosporium resinae, Homodendrum resinae, Hormoconis resinae, Cladosporium avellaneum,* die asexuellen Formen *Cladosporium resinae f. avellaneum, Cladosporium resinae f. resiane (= f. viride), Cladosporium resinae f. albidum, Cladosporium resinae f. sterile* sein, wobei Pilzarten dieser Art als Bestandteil der natürlichen Flora die in Kontakt mit dem Substrat stehende Biokohle besiedeln oder in Form einer Pilzkultur der Biokohle hinzugefügt werden.

Vorzugsweise wird für das Verfahren eine Biokohle, die aus rein pflanzlicher Biomasse und/oder verunreinigter Biomasse, wie z.B. Holzschnipseln, Holzsägespänen und oder Holzpellets, gewonnen wurde und die einen hohen Ligninanteil aufweist, eingesetzt.

Insbesondere ein hoher Lignin-Gehalt bedeutet eine gute Verträglichkeit für die genannten *Amorphothecaceae.* Vorzugsweise sollte daher die als Ausgangsmaterial gewählte Biomasse zur Herstellung der Biokohle einen Ligninanteil von über 25 Gew.-%, vorzugsweise von 27 Gew.-% bis 50 Gew.-%, besitzen.

Vorzugsweise weist die Biokohle eine spezifische Dichte zwischen 0,30 und 1,5 g/cm³ auf. Ihr Kohlenstoffgehalt liegt vorzugsweise zwischen 50 und 99,9 Gew.-%.

Dabei sollte die Biokohle eine gekörnte Form eine Siebgröße von 100 mm bis 0,1 mm besitzen.

Das Verhältnis von Biokohle zu Kontaminenten (Gewicht zu Gewicht) liegt vorzugsweise zwischen 0,01 : 1 bis 10 : 1, weiter vorzugsweise zwischen 0,1 : 1 bis 1,3 : 1.

Vorzugsweise wird bei einer Gewässer-Dekontamination mit Pilzen präparierte Biokohle mit einer spezifischen Porenstruktur und einer spezifischen Dichte <1g/cm³ als schwimmender Teppich auf einer Wasseroberfläche aufgebracht, die mit aufschwimmenden Kohlenwasserstoffen bedeckt und kontaminiert ist.

Da sich die erwünschten Mikroorganismen für die Einlagerung an die Biokohle ubiquitär im Boden befinden, wird die als Trägersubstanz genannte, granulierte Biokohle beispielsweise dem kontaminierten Boden je nach Verschmutzungsgrad in einer Menge (Gewicht zu Gewicht) von 0,1 : 1 bis 1,3 : 1 beigemischt. Bei Umgebungstemperaturen von 15°C bis 30°C kann sich der Pilz entwickeln und auf die Kontaminenten einwirken.

Die Erfindung wird anhand von Ausführungsbeispielen erläutert.

### Beispiel 1:

Mit Dieseltreibstoff kontaminierter Boden wird mit stückiger Biokohle im Siebgrößenbereich 2,3 bis 3,1mm durch geeignetes Einarbeiten, vorzugsweise Einfräsen, innig in Kontakt gebracht. Der Boden muss feucht, locker und gut belüftet sein. Ein Biokohlebedarf zwischen 10 und 100 Liter Biokohle pro m² Boden ist für das Verfahren vorteilhaft. Der Wassergehalt beträgt zwischen 90 und 15 Gew.-%. Unter 15 Gew.-% Wassergehalt ist die aerobe Vergärung durch *Cladosporium resinae* gehemmt. Der aerobe Abbau von Dieseltreibstoff durch *Cladosporium resinae* verläuft über Hydroxylierungen und Carboxylierungen der vornehmlich gesättigten Kohlenwasserstoffe letztendlich zu Wasser und Kohlendioxid. Eine Temperatur zwischen 5 °C und 45 °C ist für das Verfahren vorteilhaft. Unter 5 °C ist die aerobe Vergärung durch *Cladosporium resinae* gehemmt. Es wird der pH-Bereich zwischen 4 und 6,5 ist für das Verfahren gewählt. Unter pH 4 und über pH 6,5 ist die aerobe Vergärung durch *Cladosporium resinae* weitgehend gehemmt.

### Beispiel 2:

### Sanierung von kontaminiertem Seewasser

Mit Mineralöl kontaminiertes Seewasser wird mit stückiger Biokohle im Partikelgrößenbereich 2,3 bis 3,1mm in Kontakt gebracht, so dass sich ein aufschwimmender, möglichst zusammenhängender Biokohleteppich bildet. Abhängig vom Grad der Verschmutzung ist ein Biokohlebedarf zwischen 5 und 50 Liter Biokohle pro m² Wasserfläche für das Verfahren erforderlich. Der aerobe Abbau von Mineralöl durch *Cladosporium resinae* verläuft über Hydroxylierungen und Carboxylierungen der vornehmlich gesättigten Kohlenwasserstoffe letztendlich zu Wasser und Kohlendioxid. Eine Temperatur zwischen 5 und 45 °C ist für das Verfahren vorteilhaft. Unter 5 °C ist die aerobe Vergärung durch *Cladosporium resinae* gehemmt. Es wird der pH-Bereich zwischen 4 und 6,5 ist für das Verfahren gewählt. Unter pH 4 und über pH 6,5 ist die aerobe Vergärung durch *Cladosporium resinae* weitgehend gehemmt.

### Beispiel 3:

### Sanierung von kontaminierter Luft

Mit Dieseltreibstoff kontaminierte Luft wird in einem Festbettabsorber mit stückiger Biokohle (vorteilhafter Partikelgrößenbereich 2,3 bis 3,1 mm) so in Kontakt gebracht, dass die vom Absorber abströmende Luft einen Kohlenwasserstoffgehalt von ≤ 3 mg (Kohlenwasserstoffe)/m³ (abströmende Luft) aufweist. Die Biokohle wird mittels Schneckenförderung in den Festbettabsorber eingebracht. Die abströmende Luft wird in einer nachgeschalteten Filteranlage mit integriertem Sporenfilter von Feinstpartikeln befreit und kann dann in das Ökosystem zurückgeführt werden. Die schadstoffbeladene Biokohle wird mittels kontinuierlicher Schneckenförderung aus dem Festbettabsorber ausgebracht und in einen Gärreaktor eingebracht. Im Gärreaktor wird die schadstoffbeladene Biokohle wasserfeucht bei pH 4 bis pH 6,5 und Temperaturen zwischen 5 bis 45 °C unter ausreichender Belüftung durch *Cladosporium resinae* aerob vergoren. Der aerobe Abbau von Dieseltreibstoff durch *Cladosporium resinae* verläuft über Hydroxylierungen und Carboxylierungen der vornehmlich gesättigten Kohlenwasserstoffe letztendlich zu Wasser und Kohlendioxid. Zur Betriebssicherheit ist der Gärreaktor beheizbar und mit einem Abluftsystem mit integriertem Sporenfilter ausgestattet. Nach der Vergärung wird die Biokohle mittels Schneckenförderung aus dem Gärreaktor ausgebracht und dann in einem nachgeschalteten Autoklaven hydrothermal sterilisiert und regeneriert. Unter 5 °C und über 45 °C ist die aerobe Vergärung durch *Cladosporium resinae* gehemmt. Unter pH 4 und über pH 6,5 ist die aerobe Vergärung durch *Cladosporium resinae* gehemmt.

### Beispiel 4:

### Sanierung von kontaminiertem Oberflächenwasser

Mit Dieseltreibstoff kontaminiertes Oberflächenwasser wird in einem Festbettadsorber mit stückiger. Biokohle (vorteilhafter Partikelgrößenbereich 2,3 bis 3,1 mm) so in Kontakt gebracht, dass das vom Festbettadsorber abfließende Wasser einen Kohlenwasserstoffgehalt von ≤ 1 mg/l aufweist. Die Biokohle wird mittels Schneckenförderung in den Festbettabsorber eingebracht. Das abfließende dekontaminierte Wasser wird in einer nachgeschalteten Filteranlage von Feinstpartikeln befreit und kann dann in das Ökosystem zurückgeführt werden.

Die schadstoffbeladene Biokohle wird mittels kontinuierlicher Schneckenförderung aus dem Festbettadsorber ausgebracht und in einen Gärreaktor eingebracht. Im Gärreaktor wird die schadstoffbeladene Biokohle wasserfeucht bei pH 4 bis pH 6,5 und Temperaturen zwischen 5-45 °C unter ausreichender Belüftung durch *Cladosporium resinae* aerob vergoren. Der aerobe Abbau von Dieseltreibstoff durch *Cladosporium resinae* verläuft über Hydroxylierungen und Carboxylierungen der vornehmlich gesättigten Kohlenwasserstoffe letztendlich zu Wasser und Kohlendioxid. Zur Betriebssicherheit ist der Gärreaktor beheizbar und mit einem Abluftsystem mit integriertem Sporenfilter ausgestattet. Nach der Vergärung wird die Biokohle mittels horizontaler Schneckenförderung aus dem Gärreaktor ausgebracht und dann in einem nachgeschaltetem Autoklaven hydrothermal sterilisiert und regeneriert. Unter 5 °C und über 45 °C ist die aerobe Vergärung durch *Cladosporium resinae* gehemmt. Unter pH 4 und über pH 6,5 ist die aerobe Vergärung durch *Cladosporium resinae* gehemmt.

### Beispiel 5

### Sanierung von kontaminiertem Erdreich

Mit Mineralöl kontaminiertes Erdreich wird in einer Mischeinheit mit stückiger Biokohle (vorteilhafter Partikelgrößenbereich 2,3 bis 3,1mm) innig gemischt und zur aeroben Vergärung in eine Rottenhalle eingelagert. Abhängig vom Grad der Verschmutzung des Erdreichs mit Mineralöl ist ein Biokohlebedarf zwischen 50 und 500 Liter Biokohle pro m³ Erdreich für das Verfahren erforderlich. Das Erdreich muss feucht, locker und gut belüftet sein. Ein Wassergehalt zwischen 90 und 15 % ist für das Verfahren vorteilhaft. Unter 15 % Wassergehalt ist die aerobe Vergärung durch *Cladosporium resinae* gehemmt. In der Rottenhalle wird die schadstoffbeladene Biokohle wasserfeucht bei pH 4 bis pH 6,5 und Temperaturen zwischen 5 und 45 °C unter ausreichender Belüftung durch *Cladosporium resinae* aerob vergoren. Der aerobe Abbau von Mineralöl im Erdreich durch *Cladosporium resinae* verläuft über Hydroxylierungen und Carboxylierungen der vornehmlich gesättigten Kohlenwasserstoffe letztendlich zu Wasser und Kohlendioxid. Unter 5 °C und über 45 °C ist die aerobe Vergärung durch *Cladosporium resinae* gehemmt. Unter pH 4 und über pH 6,5 ist die aerobe Vergärung durch *Cladosporium resinae* gehemmt. Zur Betriebssicherheit ist die Rottenhalle beheizbar und mit einem Abluftsystem mit integriertem Sporenfilter ausgestattet.

## Patentansprüche

1. Verfahren zum mikrobiologischen Abbau unerwünschter Anteile von wasser- und erdreichgefährdenden Mineralölen und Mineralölprodukten, im Folgenden "Kohlenwasserstoffe" genannt, in Ecosystemen, wie limnischen und marinen Gewässern, hauptsächlich aus Wasser bestehenden Flüssigkeiten, Böden, Sedimenten, Schlämmen, Abfallgemischen und ähnlichen Substraten, wobei auf das und/oder in das jeweilige Substrat zum Abbau der in ihm enthaltenen Kohlenwasserstoffe ein festes Material, bestehend aus einem die Kohlenwasserstoffe kontaktierenden kohlenstoffhaltigen Träger mit biogenetischer Eigenschaft zur Ernährung von kohlenwasserstoffabbauenden Fungus-Arten auf- bzw. eingebracht wird, und das Material mit zur Degradierung von Kohlenwasserstoffen befähigten Fungi besetzt wird,
**dadurch gekennzeichnet,**
**dass** als Träger an sich bekannte Biokohle verwendet wird, die durch katalytische hydrothermale Carbonisierung gewonnen wurde, und als degradierende Fungi Pilze von der Familie *Amorphothecaceae* gewählt werden, wobei Pilzarten dieser Familie als Bestandteil der natürlichen Flora die in Kontakt mit dem Substrat zu bringende Biokohle besiedeln und/oder in Form einer Pilzkultur der Biokohle hinzugefügt werden.

2. Verfahren nach Anspruch 1,
**dadurch gekennzeichnet,**
**dass** der die Kohlenwasserstoffe degradierende Pilz von der Gattung *Amorphotheca* gewählt wird, wobei Pilzgattungen dieser Gattung als Bestandteil der natürlichen Flora die in Kontakt mit dem Substrat stehende Biokohle besiedeln oder in Form einer Pilzkultur der Biokohle hinzugefügt werden.

3. Verfahren nach Anspruch 1,
**dadurch gekennzeichnet,**
**dass** der die Kohlenwasserstoffe degradierende Pilz von der Art Amorphotheca resinae in seinem perfect state und/oder in seinem imperfect state und/oder in seinem Intermediat, hier im Speziellen benannt die Synonyme creosote fungus, *Cladosporium resinae, Homodendrum resinae, Hormoconis resinae, Cladosporium avellaneum,* die asexuellen Formen *Cladosporium resinae f. avellaneum, Cladosporium resinae f. resinae* (= *f. viride*), *Cladosporium resinae f. albidum*, Cladosporium resinae f. sterile gewählt wird, wobei Pilzarten dieser Art als Bestandteil der natürlichen Flora die in Kontakt mit dem Substrat stehende Biokohle besiedeln oder in Form einer Pilzkultur der Biokohle hinzugefügt werden.

4. Verfahren nach Anspruch 1, **dadurch gekennzeichnet, dass** das Verfahren in situ, d.h. nach Überführung der Biokohle und der Pilzkulturen in das verunreinigte Substrat vor Ort unter empirisch ermittelten optimierten Bedingungen, vorzugsweise bei einer Temperatur von 5° bis 45°C, durchgeführt wird.

5. Verfahren nach Anspruch 1, **dadurch gekennzeichnet, dass** das Verfahren ex situ, d.h. nach Überführung des verunreinigten Substrates in einen Reaktor zusammen mit Biokohle und Pilzkulturen, in diesem unter empirisch ermittelten optimierten Bedingungen, vorzugsweise bei einer Temperatur von 5° bis 45°C, durchgeführt wird.

6. Verfahren nach einem der vorhergehenden Ansprüche, **dadurch gekennzeichnet, dass** die Biokohle mit einer die Kohlenwasserstoffe adsorbierenden porösen Mikrostruktur ausgestattet wird.

7. Verfahren nach einem der vorhergehenden Ansprüche, **dadurch gekennzeichnet, dass** die Biokohle aus rein pflanzlicher Biomasse und/oder verunreinigter Biomasse, Holzschnitzeln, Holzsägespänen und/oder Holzpellets gewonnen wurde.

8. Verfahren nach einem der vorhergehenden Ansprüche, **dadurch gekennzeichnet, dass** die Biokohle eine spezifische Dichte zwischen 0,30 und 1,5 g/cm3 besitzt.

9. Verfahren nach einem der vorhergehenden Ansprüche, **dadurch gekennzeichnet, dass** die Biokohle einen Kohlenstoffgehalt wischen 50 und 99 Gew.-% besitzt.

10. Verfahren nach einem der vorhergehenden Ansprüche, **dadurch gekennzeichnet, dass** die Biokohle in gekörnter Form eine Siebgröße von 100 bis 0,1 mm bei Einbringung in Schlämme, Industrieabfälle oder lockere Böden als Kontaminenten besitzt.

11. Verfahren nach Anspruch 1 oder 10, **dadurch gekennzeichnet, dass** das Verhältnis von Biokohle zu Kontaminenten (Gewicht zu Gewicht) zwischen 0,01 : 1 bis 10 : 1 beträgt.

12. Verfahren nach Anspruch 11, **dadurch gekennzeichnet, dass** das Verhältnis von Biokohle zu Kontaminenten (Gewicht zu Gewicht) zwischen 0,01 : 1 bis 3 : 1 beträgt.

13. Verfahren nach Anspruch 11, **dadurch gekennzeichnet, dass** das Verhältnis von Biokohle zu Kontaminenten (Gewicht zu Gewicht) zwischen 0,1 : 1 bis 1,3 : 1 beträgt.

14. Verfahren nach einem der vorhergehenden Ansprüche, **dadurch gekennzeichnet, dass** präparierte Biokohle mit einer spezifischen Porenstruktur und einer spezifischen Dichte <1g/cm³ als schwimmender Teppich auf einer Wasseroberfläche aufgebracht wird, die mit aufschwimmenden Kohlenwasserstoffen bedeckt und kontaminiert ist.

## Claims

1. Method of microbiologically breaking down unwanted amounts of water- and soil-hazardous mineral oils and mineral oil products, referred to in the following as "hydrocarbons", in ecosystems such as limnic and marine water bodies, mainly fluids consisting mainly of water, soils, sediments, muds, waste mixtures and similar substrates, where a solid material consisting of a carbon-laden carrier making contact with the hydrocarbons and having the biogenetic property of feeding hydrocarbon-degradative species of fungus is applied to and/or inoculated into the respective substrate to break down the hydrocarbons held in it and the material is sown with fungi capable of degrading hydrocarbons,
**characterised in**
**that** biocoal, which is known per se, and that has been obtained by catalytic, hydrothermal carbonisation is used as a carrier and that fungi of the family amorphothecaceae are selected as degradative fungi, where fungus species of this family as a constituent of natural flora populate the biocoal that is to be brought into contact with the substrate and/or are added to the biocoal in the form of a fungus culture.

2. Method in accordance with claim 1,
**characterised in**
**that** the hydrocarbon-degradative fungus of the family amorphotheca is selected, where fungus species of this family as a constituent of natural flora populate the biocoal making contact with the substrate or are added to the biocoal in the form of a fungus culture.

3. Method in accordance with claim 1,
**characterised in**
**that** the hydrocarbon-degradative fungus of the species amorphotheca resinae in its perfect state and/or in its imperfect state and/or in its intermediate, here specifically also called creosote fungus, cladosporium resinae, homodendrum resinae, hormoconis resinae, cladosporium avellaneum, the asexual forms cladosporium resinae f. avellaneum, cladosporium resinae f. resinae (= f. viride), cladosporium resinae f. albidum, cladosporium resinae f. sterile is selected, where fungi of this family, as a constituent of natural flora, populate the biocoal making contact with the substrate or are added to the biocoal in the form of a fungus culture.

4. Method in accordance with claim 1, **characterised in that** the method is applied in situ, that is, after transfer of the biocoal and fungus cultures into the contaminated substrate on site under empirically determined optimised conditions, preferably at a temperature of between 5 and 45 °C.

5. Method in accordance with claim 1, **characterised in that** the method is applied ex situ, that is, after transfer of the contaminated substrate together with biocoal and fungus cultures into a reactor, under empirically determined optimised conditions, preferably at a temperature of between 5 and 45 °C.

6. Method in accordance with any one of the foregoing claims, **characterised in that** the biocoal is provided with a hydrocarbon-absorptive porous microstructure.

7. Method in accordance with any one of the foregoing claims, **characterised in that** the biocoal has been obtained from purely vegetable biomass and/or contaminated biomass, wood chips, wood shavings and/or wood pellets.

8. Method in accordance with any one of the foregoing claims, **characterised in that** the biocoal has a specific gravity of between 0.30 and 1.5 g/cm³.

9. Method in accordance with any one of the foregoing claims, **characterised in that** the biocoal has a carbon content of between 50 and 99 % by weight.

10. Method in accordance with any one of the foregoing claims, **characterised in that** the biocoal in granular form has a screen size of between 100 and 0.1 mm, when inoculated into muds, industrial wastes or loose soils as contaminants.

11. Method in accordance with claim 1 or 10, **characterised in that** the proportion of biocoal to contaminants (weight for weight) is between 0.01:1 and 10:1.

12. Method in accordance with claim 11, **characterised in that** the proportion of biocoal to contaminants (weight for weight) is between 0.01:1 and 3:1.

13. Method in accordance with claim 11, **characterised in that** the proportion of biocoal to contaminants (weight for weight) is between 0.01:1 and 1.3:1.

14. Method in accordance with any one of the foregoing claims, **characterised in that** treated biocoal with a specific pore structure and a specific gravity of < 1 g/cm³ is applied as a floating carpet to a water surface that is covered with and contaminated by floating hydrocarbons.

## Revendications

1. Procédé pour la dégradation microbiologique de fractions indésirables d'huiles minérales et de produits à base d'huile minérales dangereux pour l'eau et la terre, ci-après dénommés « hydrocarbures », dans des écosystèmes comme les eaux lacustres et marines, des liquides principalement composés d'eau, des sols, sédiments, boues, mélanges de déchets et substrats similaires, sachant que sur et/ou dans le substrat respectif est posé et/ou introduit une matière solide destinée à dégrader les hydrocarbures qu'il contient, matière composée d'un support contenant du carbone et entrant en contact avec les hydrocarbures, dotée d'une propriété biogénétique pour nourrir des types de champignons dégradant les hydrocarbures, et que la matière est colonisée par des champignons capables de dégrader des hydrocarbures,
**caractérisé en ce que**
comme support est utilisé du charbon bio en soi connu, obtenu par carbonisation catalytique hydrothermique, et que comme champignons dégradants ont été choisis des champignons de la famille des amorphothecaceæ, sachant que les types de champignons de cette famille colonisent, vu qu'il font partie intégrante de la flore naturelle, le charbon bio à amener en contact avec le substrat et/ou sont ajoutés au charbon bio sous forme de culture fongique.

2. Procédé selon la revendication 1,
**caractérisé en ce**
**que** le champignon choisi pour dégrader les hydrocarbures est du genre des amorphotheca, sachant que les champignons de ce genre, qui font partie intégrante de la flore naturelle, colonisent le charbon bio en contact avec le substrat ou sont ajoutés au charbon bio sous forme de culture fongique.

3. Procédé selon la revendication 1,
**caractérisé en ce**
**que** le champignon dégradant les hydrocarbures est de l'espèce amorphotheca resinæ choisie dans son état parfait et/ou dans son état imparfait et/ou dans son état intermédiaire, ici pour les mentionner plus spécialement, les synonymes creosote fungus, cladosporium resinæ, homodendrum resinæ, hormoconis resinæ, cladosporium avellaneum, les formes asexuées cladosporium resinæ f. avellaneum, cladosporium resinæ f. resinæ (= f. viride), cladosporium resinæ f. albidum, cladosporium resinæ f. sterile, sachant que les champignons de cette espèce, en tant que partie intégrante de la flore naturelle, colonisent le charbon bio en contact avec le substrat, ou sont ajoutés au charbon bio sous la forme d'une culture fongique.

4. Procédé selon la revendication 1, **caractérisé en ce que** le procédé in situ, c.-à-d. après avoir transféré le charbon bio et les cultures fongiques dans le substrat souillé, sur place, dans des conditions optimisées empiriquement déterminées, est exécuté à une température de préférence comprise entre 5 et 45 °C.

5. Procédé selon la revendication 1, **caractérisé en ce que** le procédé ex situ, c.-à-d. après avoir transféré le substrat souillé dans un réacteur avec le charbon bio et des cultures fongiques, est exécuté dans ce réacteur dans des conditions optimisées empiriquement déterminées, à une température de préférence comprise entre 5 et 45 °C.

6. Procédé selon l'une des revendications précédentes, **caractérisé en ce que** le charbon bio présente une microstructure poreuse adsorbant les hydrocarbures.

7. Procédé selon l'une des revendications précédentes, **caractérisé en ce que** le charbon bio est extrait d'une biomasse purement végétale et/ou d'une biomasse contaminée, de plaquettes de bois, de copeaux de sciage du bois et/ou de pellets de bois.

8. Procédé selon l'une des revendications précédentes, **caractérisé en ce que** le charbon bio possède une densité spécifique comprise entre 0,30 et 1,5 g/cm³.

9. Procédé selon l'une des revendications précédentes, **caractérisé en ce que** le charbon bio présente une teneur en carbone comprise entre 50 et 99 % pondéraux.

10. Procédé selon l'une des revendications précédentes, **caractérisé en ce que** le charbon bio sous forme de grain présente une granulométrie tamisée comprise entre 100 et 0,1 mm, lorsque introduit dans des boues, des déchets industriels ou des sols meubles en tant que contaminants.

11. Procédé selon la revendication 1 ou 10, **caractérisé en ce que** le rapport (poids : poids) entre le charbon bio et les contaminants est compris entre 0,01 : 1 et 10 : 1.

12. Procédé selon la revendication 11, **caractérisé en ce que** le rapport (poids : poids) entre le charbon bio et les contaminants est compris entre 0,01 : 1 et 3 : 1.

13. Procédé selon la revendication 11, **caractérisé en ce que** le rapport (poids : poids) entre le charbon bio et les contaminants est compris entre 0,1 : 1 et 1,3 : 1.

14. Procédé selon l'une des revendications précédentes, **caractérisé en ce que** le charbon bio préparé, présentant une structure poreuse spécifique et une densité spécifique < 1 g/cm³ est appliqué sous forme de tapis flottant sur une surface d'eau recouverte et contaminée par des hydrocarbures flottants.
